# EUROPEAN PATENT APPLICATION

(11) **EP 1 000 605 A2**
(43) Date of publication of application: **17.05.2000**
(21) Application number: 99500198.9
(22) Date of filing: 27.10.1999
(51) Int. Cl.: A61K 7/00, A61K 7/50, A61K 7/48

(54) **Skin-cleaning towels impregnated with liposomes containing compositions**

(30) Priority: 28.10.1998 ES 9802260
(71) Applicant: Julia-Capmany Font, Maria Luisa, 08022 Barcelona (ES); Magrina Veciana, Maria Luisa, 08022 Barcelona (ES); Perez Lopez, Montserrat, 08022 Barcelona (ES)
(72) Inventor: Gonzalez Ensenat, Pedro, 08022 Barcelona (ES)
(74) Representative: Canadell-Isern, Roberto

(57) **Abstract**

The product is based on a liposomal suspension to which one or more specific active, cosmetic or ethic products may be added to obtain a certain complementary effect or not of the cleaning:cleaning+care;cleaning-anti-acne;
cleaning+desinfection, etc.

The product uses a towel impregnated with a suitable amount of said product, packed inside a one-dosage container, as an application support.

Likewise, the product composed of suspended liposomal structuces may be made in a solution or other Galenic form and packed in one-dosage or multi-dosage containers that include or not a towel or other application support.

The product is applied when cleaning the skin and facial tissue as an advantageous replacement, partial or total, of dissolvents, tensoactives, detergents or other dispersers made in the normal way.

## Description

### FIELD OF THE INVENTION

This Patent refers to a product for cleaning the skin and facial tissue, which replaces partially or totally dissolvents, tensoactives or detergents and other dispersers, made in their normal ways, by suspended liposomal structures.

### HISTORY

At present and as a reference to the state-of-the-art, it should be mentioned that products meant for cleaning the skin and facial tissue, basically composed of dissolvents, detergents or tensoactives and other dispersing products, in different Galenic ways of presentation which, with use, cause a large number of irritations, wounds, allergies and other adverse reactions.

Although the "cleaning" effect is noteworthy, their undesired effects subtract value from this efficiency.

### SUMMARY

The purpose of this invention is to replace, partially or totally, the products used today for cleaning the skin with suspended liposomal structures.

The main "idea" is the result of the experience in the care and treatment of the skin and facial tissue, together with the replacement of dissolvents, detergents or tensoactives or other dispersing agents for the textile industry, tanning, phytosanitary, perfumes, etc.

The developed product is based mainly on using the large dissolvent-disperser capacity that the suspended liposomal structures have to replace the products and ways used at present for bodily cleaning.

On the contrary to the already existing products, its composition and structure not only does not damage the skin and facial tissue, but it has "per se" regenerating, nutritive and hydrating properties that help in the care of the areas treated.

One or more specific active cosmetic or ethic products can be added to the liposomal suspension to obtain a certain "complementary" effect or not of the cleaning. For example: cleaning + care; cleaning + antiacne; cleaning + disinfection.

In the product that has been developed for demonstrating this invention, make-up remover-cleaning towel, a glycolic chamomile extract has been added in order to obtain an anti-inflammatory-clearing-relaxing effect, as well as borage oil to improve its nutritive-regenerating capacity.

During this development different types of liposomes have also been tested for their method of manufacture and composition and the efficiency of the different types has been able to be verified. Liposomal suspensions prepared with different manufacturing techniques have been tested, such as:
- Liposomes manufactured by Dianorm, GMBH
- Liposomes manufactured by NATTERMANN GMBH
- Liposomes manufactured by SONICACION, EXTRUSION
- Liposomal suspensions of varying composition and lipid quality have also been tested, such as:
- Liposomes prepared with 94-96% soya lecithin
- Liposomes prepared with 75-80% Soya lecithin
- Liposomes prepared with 45-50% Soya lecithin
- Liposomes prepared with 93-96% egg lecithin

Different concentrations of total lipid amounts in the final liposomal suspension have also been tested. These have varied between 25 gr/l and 0.5 gr/l.

Facial cleaning was chosen due to its greater demonstrative difficulty and commercial interest.

In all the tests the cleaning activity, as well as the texture and sensation of well-being, have been valued and compared, as well as with the products existing in the market today. All this has been done in order to demonstrate the efficiency of the invention, as well as to optimize one of its applications (the make-up remover, cleaning and treating towel for face and eyes).

In order to test said product, a one-dosage support in the form of a towel impregnated with a suitable amount of the product, placed inside an envelope or individual bag, was used.

For this reason different types of towels were tested and the compatibility with the liposomal suspension as well as the cleaning efficiency and its qualities on touch studied. The final product was stressed to verify that it did not lose any of its properties and for expiry purposes.

The final product has been dermatologically tested by PATCH-TEST and PHOTO-PATCH TEST on volunteers where each of the components of this formula has been patched, as well as the final product. The object of the epicutaneous tests, patch tests, is to demonstrate the existence of a possible allergy due to contact and thus to be able to prove the harmlessness of certain new products and substances.

These are carried out by placing products suspicious of causing allergy on the unharmed skin of the subjects in the suitable concentrations of each substance. If this causes the appearance of an eczematous wound the conclusion is that this person suffers a contact allergy to the applied substance.

Material and method: The vehicle used for the majority of substances is Vaseline. The patch test requires a piece of filter paper of 1 cm² approximately, attached to the center of a slightly bigger piece of waterproof inert material. A commercial model already exists and this has been used. It is called: True test.

The patches are placed on the back or on the outside part of the arms. Before application the skin should not be cleaned with soap or dissolvent. Once the substances are individually placed on each patch, they are stuck on the skin of the volunteers and are not removed until 48 hours later. Then, a first reading is made observing if there have been any changes in the cutaneous surface bearing the patch. A second reading is made 48 hours later (96 hours after placing the patches), making sure that the individual has not washed the area during this time. The interpretation is made in the following way:
- +?: Doubtful reaction: only a slight erythema
- +: Weak positive reaction: erythema and formation of papula
- ++: Strong positive reaction: erythema, papula and vesicle
- +++: Intense positive reaction: formation of blisters
- -: Negative reaction
- - IR: Irritating reaction

A true positive reaction to the eipcutaneous test indicates that this person is sensitive to the tested product suspicious of causing allergy.

Photo-Patch-Test: There are substances that can cause contact dermatitis when exposed to the light and for this reason the following tests are carried out: the Photo-Patch-Test which is carried out in the same way as described above and, in addition, the patched areas are exposed to an ultraviolet radiation with the wave length of more than 320 MN.

In this study each of the ingredients have been patched separately, as well as the final mixture: the towel used when dry and soaked.

50 volunteers of both sexes between the ages of 14 and 70 have been patched.

None of them presented any reaction at all and all the tests carried out were negative.

The efficiency study was made on 50 volunteers between the ages of 20 and 70, normal consumers of make-up and make-up removers, who were told about the purpose of the study and who were given 7 envelopes and "an answer sheet". The volunteers were asked to reply to the comparative questions as regards the system normally used by them, where both their objective cleaning effects (much better, better, the same, worse, much worse), treatment effects; clearing, hydration, as well as the most subjective qualities of texture, well-being and comfort were valued.

The result of this study can be summarized by concluding that only 4% (2 volunteers) considered their normal product better than the proposed alternative.

The product for cleaning the skin and facial tissue, composed of suspended liposomal structures, can also be obtained in a solution or any other Galenic form.

Likewise, this product can be packed both in one-dosage or multi-dosage containers, which include or not a towel or other application support.

In order to complement the following description and for the purpose of helping to better understand the characteristics of the invention, this descriptive report includes a set of drawings in which the most significant details of the invention have been represented in an illustrative and unlimited way:

### BRIEF DESCRIPTION OF THE DRAWINGS

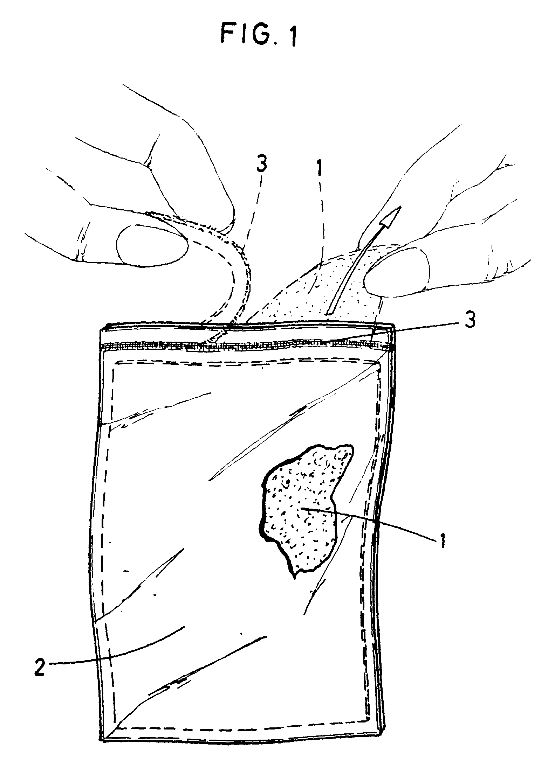

Figure 1 shows a partially sectioned view of a possible packing system of the product for cleaning the skin and facial tissue.

### DESCRIPTION OF A PERFORMANCE ACCORDING TO THE INVENTION

The following examples with an illustrative but not limited nature are detailed below:

### Example 1

Bearing in mind each and all of the mentioned principles, the make-up remover towel is composed of a liposomal suspension that contains:
53.5 gr/l of a liposomal concentrate of Soya lecithin in the form of capsules.
   Borage oil.
35.7 gr/l of chamomile glycolic extract
35.7 gr/l of propylene glycol
5 gr/l of phenoxethanol
0.53 gr/l of perfume
871.4 gr/l of deionized water

In this case the liposomal concentrate used is from the Dianorm company and contains:
100 gr/l of Soya lecithin with 94-96% of fosfatidilcolina(??)
5 gr/l of 1st pressure borage oil with a high content in linoléhico (??)
0.25 gr/l of d-l-tocopherols (natural vitamin E)

In order to prepare the final product it is necessary to unit and shake these components. This preparation should be prepared at ambient temperature.

In this respect, the chamomile glycolic extract, the propylene glycol, the phenoxethanol and the perfume are mixed simply by shaking. Once a smooth mixture is obtained, water is added and it once again shaken. Lastly, the liposomal concentrate is added.

The towel is then impregnated with the product thus obtained in a suitable amount and is placed in its individual bag.

### Example 2

Another product developed with this invention is for cleaning and caring for the hands.

This product, the same as the others, has been invented using the following philosophy: cleaning - care of the skin - hydration - comfort.

Its composition is based on:
32 gr per liter of liposomes with aloe vera oil
25 gr per liter of Asian spider crab extract
5 gr per liter of urea
5 gr per liter of 2 phenoxi-ethanol

The liposomes with aloe vera oil are a product to reinforce the hydrating re-epithelial effect natural to the liposomes and is very efficient in reinforcing and hydrating both the hands as well as nails and cuticle.

The Asian spider crab extract is a product that acts as a skin regenerator.

The urea is a product that is used to reinforce the hydrating effect.

It has been tested on 80 adult volunteers of both sexes the ages of which are between 15 and 80 years.

As regards the questionnaire for the volunteers on the product, the cleaning activity has been valued, as well as the texture of the skin, nails and cuticle at the moment of its application.

The tests were open and could not be compared with other market products as there are none with these characteristics.

The final product has been dermatologically tested by the PATCH-TEST and PHOTO-PATCH-TEST methods on the 80 volunteers mentioned above and according to the method previously described in detail.

This product is packed in one-dosage towels, in the form of a towel impregnated with a suitable amount of the product and placed inside an envelope or individual bag. In this respect, different types of towels were tested and the compatibility with the liposomal suspension studied, as well as its cleaning efficiency and qualities on contact. The final product was stressed to check that it did not lose any of its properties for the purposes of expiry.

### Example 3

Another product developed with this invention is a preparation for intimate hygiene.

This product, the same as the product of example 1, has been invented based on the cleaning philosophy - hygiene - care of the skin - comfort.

Its composition is composed of:
Miglyol oil liposomes, 25 gr per liter
Chlorhexidine Digluconate 1 gr per liter
Salt solution 973.5 gr per liter (Na Cl 3 gr per liter)

Miglyol is the trade name of what is normally known as fat acid triglycerides of a convenient average chain.

The Miglyol oil liposomes are a product that has a great cleaning action and at the same time as it hydrates and regenerates the skin.

Chlorhexidine digluconato (??): This serves as a good aseptic to obtain a perfect intimate hygiene.

In this case the efficiency tests were carried out on 65 adult volunteers between the ages of 16 and 79 years.

A one-dosage support was used to test this product, in the form of a towel impregnated with a suitable amount of the product and placed inside an envelope or individual bag, and another dry towel placed inside another individual bag.

In the questionnaire given to the volunteers on the product, emphasis was put on the tolerance of the two towels, as well as on the great cosmetic acceptability due to their use in intimate areas.

In this respect, different types of towels were tested and the compatibility with the liposomal suspension studied, as well as its cleaning efficiency and qualities on contact.

The final product was stressed to verify that it did not lose any of its properties.

The tests were open and could not be compared with other market products as there are none with these characteristics.

The final product has been dermatologically tested by the PATCH-TEST and PHOTO-PATCH-TEST methods on the 80 volunteers mentioned above and according to the method previously described in detail.

In Figure 1 the product -1- for cleaning the skin and facial tissue can be seen, which is composed of suspended liposomal structures that can also be obtained in solutions or other possible forms.

This product -1-, which in this specific case where it is merely shown for the purpose of example in figure 1, is placed inside a one-dosage container consisting of an envelope -2-which is hermetically closed and is able to be opened along a slit -3-, although other container forms have not been discarded, as well as its impregnation on a towel or other application support.

## Claims

1. PRODUCT FOR CLEANING THE SKIN AND FACIAL TISSUE which replaces partially or totally dissolvents, tensoactives or detergents and other dispersers, made in their normal ways, basically characterized by the fact that it is composed of liposomal structures suspended in products for cleaning the skin and facial tissue, which used a towel impregnated with a suitable amount of said product as an application support and which is packed in an individual bag.

2. PRODUCT FOR CLEANING THE SKIN AND FACIAL TISSUE, according to claim 1, characterized by the fact that its composition is based on:
53.5 gr/l of a Soya lecithin liposomal concentrate in the form of capsules
Borage oil
35.7 gr/l of chamomile glycolic extract
35.7 gr/l of propylene glycol
5 gr/l of phenoxethanol
0.53 gr/l of perfume
871.4 gr/l of deionized water

3. PRODUCT FOR CLEANING THE SKIN AND FACIAL TISSUE, according to claim 1, characterized by the fact that its composition is based on:
32 gr per liter of liposomes with aloe vera oil
25 gr per liter of Asian spider crab extract
5 gr per liter of urea
5 gr per liter of 2 Phenoxi ethanol

4. PRODUCT FOR CLEANING THE SKIN AND FACIAL TISSUE, according to claim 1, characterized by the fact that its composition is based on:
Miglyol oil liposomes, 25 gr per liter
Chlorhexidine Digluconate 1 gr per liter
Salt solution 973.5 gr per liter (Na Cl 3 gr per liter)

5. PRODUCT FOR CLEANING THE SKIN AND FACIAL TISSUE, according to claim 1, characterized by the fact that the product, composed of suspended liposomal structures, can be made into a solution or another Galenic form and can be packed in one-dosage or multi-dosage containers that include or not a towel or other application support.
